# EUROPÄISCHE PATENTANMELDUNG

(11) **EP 4 390 891 A1**
(43) Veröffentlichungstag der Anmeldung: **26.06.2024**
(21) Anmeldenummer: 22215363.7
(22) Anmeldetag: 21.12.2022
(51) Int. Cl.: G08B 21/14, G01N 33/00, G06Q 10/0631, B23K 9/00

(54) **VERFAHREN UND VORRICHTUNG ZUR ERFASSUNG DER SCHADSTOFFBELASTUNG EINER BESTIMMTEN PERSON IN DER UMGEBUNG ZUMINDEST EINER BEARBEITUNGSVORRICHTUNG ZUR DURCHFÜHRUNG VON BEARBEITUNGSPROZESSEN AN WERKSTÜCKEN**

(71) Anmelder: FRONIUS INTERNATIONAL GmbH, 4643 Pettenbach (AT)
(72) Erfinder: FRIEDL, Helmut, 4643 Pettenbach (AT); SCHÖRGHUBER, Manfred, 4643 Pettenbach (AT)
(74) Vertreter: SONN Patentanwälte GmbH & Co KG

(57) **Zusammenfassung**

Die Erfindung betrifft ein Verfahren und eine Vorrichtung (1) zur Erfassung der Schadstoffbelastung einer bestimmten Person (B) in der Umgebung zumindest einer Bearbeitungsvorrichtung (20) zur Durchführung von Bearbeitungsprozessen (BPᵢ) an Werkstücken (W) durch aufgrund der Bearbeitungsprozesse (BPᵢ) auftretende Schadstoffemissionen (Sᵢ(x,t)). Zur verlässlichen Abschätzung der auf die Person (B) wirkenden Schadstoffbelastung ist zumindest ein Sensor (4) zur Ermittlung der Position (x_{B}(t)) der Person (B) in Abhängigkeit der Zeit (t), weiters eine Datenbank (3) zum Speichern von Schadstoffemissionen (Si' (x,t)) in Abhängigkeit der durchgeführten Bearbeitungsprozesse (BPᵢ), und eine mit dem zumindest einen Sensor (4) und der Datenbank (3) verbundene Verarbeitungseinrichtung (2) vorgesehen, welche
Verarbeitungseinrichtung (2) zur Abschätzung der auf die Person (B) wirkenden Schadstoffemissionen (S_{B}(x,t)) in Abhängigkeit der ermittelten Position (x_{B}(t)) der Person (B) und anhand der in Abhängigkeit der Bearbeitungsprozesse (BPᵢ) abgerufenen Schadstoffemissionen (Si' (x,t)) aus der Datenbank (3) ausgebildet ist.

## Beschreibung

Die Erfindung betrifft ein Verfahren zur Erfassung der Schadstoffbelastung einer bestimmten Person in der Umgebung zumindest einer Bearbeitungsvorrichtung zur Durchführung von Bearbeitungsprozessen an Werkstücken durch aufgrund der Bearbeitungsprozesse auftretende Schadstoffemissionen.

Weiters betrifft die Erfindung eine Vorrichtung zur Erfassung der Schadstoffbelastung einer bestimmten Person in der Umgebung zumindest einer Bearbeitungsvorrichtung zur Durchführung von Bearbeitungsprozessen an Werkstücken durch aufgrund der Bearbeitungsprozesse auftretende Schadstoffemissionen.

Die Erfindung betrifft ganz allgemein Bearbeitungsvorrichtungen zur Durchführung von Bearbeitungsprozessen an Werkstücken, bei welchen aufgrund der Bearbeitungsprozesse Schadstoffe emittiert werden. Üblicherweise befinden sich mehrere Bearbeitungsvorrichtungen zur Durchführung verschiedener Bearbeitungsprozesse an verschiedenen Werkstücken in einer Produktionshalle, in welcher sich auch mehrere Personen aufhalten und entsprechend bewegen. Natürlich kann die Schadstoffbelastung mehrerer Personen, welche sich in der Umgebung einer Vielzahl von Bearbeitungsvorrichtungen mit parallel ablaufenden Bearbeitungsprozessen ausgesetzt sind, parallel erfasst werden. Die Schadstoffe können beispielsweise beim Lackieren von Werkstücken, bei anderen Behandlungen der Oberfläche von Werkstücken oder bei Füge- oder Trennverfahren, bei welchen Werkstücke thermisch oder mechanisch bearbeitet werden, entstehen. Insbesondere ist die vorliegende Erfindung auf Lichtbogenbearbeitungsvorrichtungen zur Durchführung von Lichtbogenprozessen gerichtet. Ganz besonders betrifft die vorliegende Erfindung Schweißvorrichtungen zur Durchführung von Schweißprozessen.

Die beim Bearbeitungsprozess entstehenden Schadstoffe können von Personen, welche sich in der Umgebung des Bearbeitungsprozesses aufhalten, aufgenommen werden und diesen Personen schaden. Bei den Personen muss es sich nicht zwingend um die Bediener der Bearbeitungsvorrichtungen handeln, sondern auch andere Personen, welche sich beispielsweise in der gleichen Produktionshalle aufhalten und andere Tätigkeiten verrichten und durch Schadstoffe gefährdet werden können. Dass es sich bei der Person um eine "bestimmte" Person handelt, soll zum Ausdruck bringen, dass eine Zuordnung dieser Person, deren Schadstoffbelastung erfasst wird, stattfinden muss. Wie und wann diese Zuordnung stattfindet, ist für die vorliegende Erfindung unerheblich. Es muss lediglich eine Zuordnung jener Person, deren Schadstoffbelastung erfasst werden soll, mit der Person deren Position ermittelt wird, ermöglicht werden. Als bestimmte Person kann auch eine fiktive Person herangezogen werden, deren Position innerhalb der Produktionshalle angenommen wird und deren fiktive Schadstoffbelastung erfasst wird. Auf diese Weise kann die Gefährlichkeit einer Produktionshalle abgeschätzt und die fiktive Schadstoffbelastung erfasst werden. Wenn diese unzulässig hoch ist, kann die Produktionshalle für den Zutritt von Personen gesperrt werden und eine Lüftung erzwungen werden.

Beim Schweißen entsteht prozessbedingt, insbesondere durch die auftretenden hohen Temperaturen aufgrund des Lichtbogens, eine Vielzahl von festen und gasförmigen Schadstoffen, welche die Gesundheit der Schweißer oder anderer Personen negativ beeinflussen können. Beispielsweise treten beim Schweißen Feinstaub, Ozon, Rauch, etc. auf. Für den menschlichen Körper gehört Feinstaub zu den gefährlichsten Schadstoffen. Aufgrund ihrer geringen Größe im Bereich einiger µm können die Partikel in die Lungenbläschen eindringen und die Gesundheit schädigen, indem sie beispielsweise Asthmaanfälle hervorrufen oder zu Herz-Kreislauf-Erkrankungen beitragen.

Zum Schutz der Personen im Bereich von Bearbeitungsvorrichtungen wurden Belastungsgrenzwerte entwickelt, welche von den Betroffenen nicht überschritten werden dürfen. Mit Hilfe zeitabhängiger MAK (Maximale Arbeitsplatz Konzentration)-Werte wird beispielsweise ein Durchschnittswert der Schadstoffbelastung über einen vorgegebenen Zeitraum festgelegt. So gibt der Grenzwert MAK TGG-8 die durchschnittliche Schadstoffbelastung über 8 Stunden oder der Wert MAK TGG-15 den Durchschnittswert über einen kürzeren Zeitraum von 15 Minuten an. Es gibt auch MAK-C Werte (Ceiling = Höchstwert), welche eine absolute Belastungsgrenze angeben, die nicht überschritten werden dürfen.

Zum Schutz von Schweißern sind Schweißhelme bekannt geworden, welche den Träger vor gefährlichen Konzentrationen von Schadstoffen warnen. Beispielsweise beschreibt die EP 2 696 823 B1 eine Konstruktion eines solchen Schweißhelms mit einem Sensor zur Überwachung von Schweißdämpfen und einer integrierten Warnvorrichtung, welche dem Schweißer anzeigen, dass vorgegebene Grenzwerte überschritten wurden und beispielsweise eine Pause zur Regeneration eingelegt werden muss.

Aus der WO 2015/036652 A1 geht ein Schweißhelm hervor, der mit einem am Rücken angeordneten Gerät zur Versorgung mit Frischluft verbunden ist, welches den Schweißer insbesondere in beengten Räumen im Falle der Überschreitung gefährlicher Grenzwerte von Kohlenstoffdioxid schützt.

Derartige vom Schweißer zu tragende Schutzvorrichtungen stellen eine Einschränkung von Wahrnehmung der Umgebung, Beweglichkeit und Flexibilität dar. Zusätzliche Geräte bedeuten zusätzliches Gewicht sowie zusätzliche Kabel. Diese werden von den Schweißern speziell in engen Räumen aus sicherheitstechnischen Gründen meist nicht akzeptiert.

Neben solchen von den betroffenen Personen zu tragenden Schutzvorrichtungen sind auch Einrichtungen bekannt, welche die Luft in den Produktionshallen verbessern und dadurch die Schadstoffkonzentrationen senken. Unter derartige Einrichtungen fallen Absaugeinrichtungen allenfalls mit Filtern und Leitungen zur Frischluftzufuhr. Zur Regelung der Absaugung und Luftzufuhr ist es notwendig, die Schadstoffbelastung an repräsentativen Stellen in der Produktionshalle zu messen. Die tatsächliche Belastung von Personen, welche sich innerhalb der Produktionshalle bewegen, kann auf diese Weise nicht ermittelt werden. Der Aufwand ist dennoch sehr hoch und erfordert in der kühlen Jahreszeit einen zusätzlichen Energieaufwand für das Beheizen der Produktionshalle.

Darüber hinaus sind auch Personen, welche nicht unmittelbar an der Bearbeitungsvorrichtung tätig sind und beispielsweise Werkstücke nachbearbeiten oder transportieren, zu einem gewissen Grad den Schadstoffen in der Produktionshalle ausgesetzt. Die Schadstoffbelastung dieser Personen wird in der Regel nicht oder nur zu Zeiten der Bearbeitung an der Bearbeitungsvorrichtung ermittelt, weshalb die reale Belastung dieser Personen nicht oder nicht genau erfasst werden kann.

Die Aufgabe der vorliegenden Erfindung besteht daher in der Schaffung eines oben genannten Verfahrens und einer Vorrichtung zur Erfassung der Schadstoffbelastung einer bestimmten Person in der Umgebung zumindest einer Bearbeitungsvorrichtung zur Durchführung von Bearbeitungsprozessen an Werkstücken durch aufgrund der Bearbeitungsprozesse auftretende Schadstoffemissionen, durch welche die real auftretende Schadstoffbelastung für diese Person möglichst genau abgeschätzt werden kann, sodass vor einer allenfalls auftretenden Gesundheitsschädigung der Person rechtzeitig entsprechende Maßnahmen getroffen werden können. Die Schadstoffbelastung der Person soll außerdem möglichst gering sein und die Person soll in ihrer Mobilität und Wahrnehmung möglichst wenig eingeschränkt werden. Der Aufwand soll möglichst gering und somit kostengünstig sein. Nachteile bekannter Verfahren und Vorrichtungen sollen verhindert oder zumindest reduziert werden.

Gelöst wird die erfindungsgemäße Aufgabe in verfahrensmäßiger Hinsicht dadurch, dass die Position der Person in Abhängigkeit der Zeit ermittelt wird, weiters in einer Datenbank in Abhängigkeit der durchgeführten Bearbeitungsprozesse gespeicherte Schadstoffemissionen abgerufen werden und die Schadstoffbelastung der Person dadurch erfasst wird, dass die auf die Person wirkenden Schadstoffemissionen in einer Verarbeitungseinrichtung in Abhängigkeit der ermittelten Position der Person und anhand der aus der Datenbank abgerufenen gespeicherten Schadstoffemissionen in Abhängigkeit der durchgeführten Bearbeitungsprozesse abgeschätzt werden. Erfindungsgemäß wird die Schadstoffbelastung einer bestimmten Person dadurch erfasst, dass in Abhängigkeit des Aufenthaltsorts der Person innerhalb der Umgebung der zumindest einen Bearbeitungsvorrichtung einerseits und in Abhängigkeit der durchgeführten Bearbeitungsprozesse anhand von Erfahrungswerten, beispielsweise aus Versuchs-Bearbeitungsprozessen, die dabei auftretenden Schadstoffemissionen, andererseits die Schadstoffbelastung der Person abgeschätzt wird. Somit wird die Schadstoffbelastung ohne die Notwendigkeit der Installation aufwändiger Sensoren anhand von gespeicherten Erfahrungswerten abgeschätzt. Die jeweilige Person braucht keine schweren Schutz- oder Messeinrichtungen tragen und ist dadurch nicht in ihrer Mobilität eingeschränkt. Durch die relativ genaue Einschätzung der Schadstoffbelastung der jeweiligen Person(en) können Maßnahmen zur Verbesserung der Umgebungsluft im Bereich der Bearbeitungsvorrichtung(en) an die tatsächlichen Gegebenheiten optimal angepasst werden und somit Energie für die Belüftung und allenfalls Heizung der Produktionshalle gespart werden. Die Position der bestimmten Personen kann relativ einfach, beispielsweise über Indikatoren, insbesondere über Sensoren an der Kleidung, Mobiltelefone, Smartwatches, oder dgl. ermittelt werden. Das erfindungsgemäße Verfahren trägt dazu bei, dass die Personen in der Umgebung der Bearbeitungsvorrichtungen rechtzeitig vor dem Überschreiten vorgegebener Grenzwerte bestimmter Schadstoffe eine Pause einlegen und somit keine gesundheitlichen Schäden erleiden. Natürlich kann das Verfahren zur parallelen Erfassung der Schadstoffbelastung mehrerer Personen in der Umgebung mehrerer (auch verschiedener) Bearbeitungsvorrichtungen herangezogen werden, wie es in einer großen Produktionshalle im Allgemeinen der Fall sein wird.

Die gespeicherten Schadstoffemissionen können beispielsweise in Abhängigkeit des Materials der bearbeiteten Werkstücke und bzw. oder der Art der Bearbeitungsprozesse und bzw. oder Parametern der Bearbeitungsprozesse, beispielsweise der Leistung der Bearbeitungsprozesse, und bzw. oder der Stabilität der Bearbeitungsprozesse abgerufen werden. Je nach Bearbeitungsprozess können die angeführten Eigenschaften oder Parameter wesentlichen Einfluss auf die Schadstoffemissionen haben. Beim Schweißen beispielsweise wird die Konzentration der entstehenden Schadstoffe stark von der Schweißleistung und der Stabilität des Schweißprozesses bestimmt. Die Leistung des Bearbeitungsprozesses kann in der Regel einfach ermittelt werden, beispielsweise über den Schweißstrom bei einem Schweißprozess. Bei stabilen Prozessen ist die Schadstoffemission deutlich geringer als bei instabil ablaufenden Prozessen. Wie die Stabilität des Bearbeitungsprozesses gemessen wird, hängt von der Art des jeweiligen Bearbeitungsprozesses ab. Beispielsweise kann bei einem Kurzschlussbehafteten Schweißprozess die Stabilität durch Messung der Zeiten der Kurzschlussphasen und Lichtbogenphasen bzw. der resultierenden Schweißfrequenz festgestellt und die entsprechenden gespeicherten Schadstoffemissionen für die Abschätzung der Schadstoffbelastung der bestimmten Person herangezogen werden.

Die Position der Person kann mit zumindest einem an der Person angeordneten Sensor, beispielsweise einem Mobiltelefon, und bzw. oder mit zumindest einem in der Umgebung der Person angeordneten Sensor, beispielsweise einer Kamera, ermittelt werden. Für die Anordnung von Sensoren an den Personen können Ausrüstungsgegenstände oder Kleidungsstücke, wie zum Beispiel Schweißschirme, Handschuhe oder Schuhe dienen, in oder an welchen eindeutige Codes, wie RFID (radio-frequency identification)-Codes, Strichcodes oder dgl. angeordnet werden, welche über Detektoren in den Bearbeitungsvorrichtungen oder in der Produktionshalle ausgelesen werden können. Beispielsweise kann auch ein Detektor in der Bearbeitungsvorrichtung, beispielsweise der Schweißstromquelle oder dem Schweißbrenner, die Nähe der mit einem RFID-Transponder ausgestatteten Person und somit dessen Position feststellen. Auch andere von den Personen getragene Gegenstände, wie zum Beispiel Mobiltelefone oder Smartwatches, können zur Bestimmung der Position verwendet werden und noch andere für die Schadstoffbelastung möglicherweise relevante Daten (zB. Puls, Sauerstoffsättigung, Atemfrequenz) liefern. Auch ohne spezielle Sensoren oder dgl. am Körper der bestimmten Personen kann die Position innerhalb der Produktionshalle ermittelt werden, beispielsweise über Videoüberwachung. Die Zuordnung zu den Personen kann beispielsweise über Gesichtserkennung oder andere eindeutige Identifikatoren erfolgen.

Sofern die Bearbeitungsvorrichtung(en) mobil sind, also ihre Position geändert werden kann, ist es von Vorteil, wenn auch die Position der zumindest einen Bearbeitungsvorrichtung ermittelt und bei der Abschätzung der Schadstoffbelastung der Person berücksichtigt wird. Ändert sich die Position der zumindest einen Bearbeitungsvorrichtung, so kann sich auch der Abstand zu der Person, deren Schadstoffbelastung erfasst werden soll, ändern und somit die Konzentration der von diesen Bearbeitungsvorrichtungen ausgehenden Emissionen der Schadstoffe.

An zumindest einer vorgegebenen Position können mit zumindest einem weiteren Sensor die Schadstoffemissionen gemessen und bei der Abschätzung der Schadstoffbelastung der Person berücksichtigt werden. Durch die Messung der realen Konzentrationen bestimmter Schadstoffe in der Umgebung der Bearbeitungsvorrichtungen kann die Erfassung der Schadstoffbelastung bestimmter Personen in der Umgebung der Bearbeitungsvorrichtungen noch genauer durchgeführt werden, ohne dass der Aufwand dafür zu groß ist. Die Anzahl und die Art der Sensoren für die Messung der tatsächlichen Konzentrationen bestimmter Schadstoffe wird natürlich an die Größe der Produktionshalle und die Art der Bearbeitungsvorrichtungen und somit die Art der bei den Bearbeitungsprozessen entstehenden Schadstoffe entsprechend angepasst. Die Messergebnisse von diesem zumindest einen weiteren Sensor können ebenso ausgewertet und bzw. oder in der Datenbank gespeichert werden.

Gemäß einem weiteren Merkmal des Verfahrens wird die Schadstoffbelastung der Person dadurch erfasst, indem die auf die Person wirkenden abgeschätzten Schadstoffemissionen mit der Zeit aufsummiert werden. Auf diese Weise kann die auf die jeweilige Person wirkende Dosis an Schadstoffen bestimmt werden. Üblicherweise regeneriert sich der Körper wieder, wenn er keinen Schadstoffen ausgesetzt ist, beispielsweise während Arbeitspausen. Diese Regeneration kann dadurch berücksichtigt werden, dass die aufsummierten Werte in Abhängigkeit der Zeit wieder reduziert werden, wenn die bestimmte Person keiner Schadstoffemission ausgesetzt ist.

Vorteilhafter Weise wird in Abhängigkeit der erfassten Schadstoffbelastung der Person eine Warnung ausgegeben und bzw. oder gespeichert. Dadurch kann der Person und bzw. oder einer übergeordneten Stelle angezeigt werden, wann ein absoluter Grenzwert der auf die Person wirkenden Schadstoffemissionen oder ein Grenzwert von über bestimmte Zeitspannen kumulierten auf die Person wirkenden Schadstoffemissionen überschritten wurden, und so eine Gesundheitsgefährdung ausgeschlossen oder zumindest reduziert werden. Durch das Speichern der Daten können diese auch für spätere Analysen dokumentiert werden. Natürlich können auch die Werte der ermittelten Schadstoffemissionen der bestimmten Person und bzw. oder einer übergeordneten Zentrale angezeigt werden.

Wenn in Abhängigkeit der erfassten Schadstoffbelastung der Person zumindest ein Lüfter zur Zufuhr von Frischluft und bzw. oder zur Luftzirkulation und bzw. oder zur Absaugung geregelt wird, kann die Belüftung einer Produktionshalle an die tatsächliche Gefährdung der Personen angepasst werden. Dadurch kann bei niedrigeren Schadstoffbelastungen Energie für die Belüftung und bzw. oder Heizung der Produktionshalle gespart werden. Natürlich kann bei Anordnung mehrerer Lüfter die Menge und auch der Ort der Absaugung bzw. Zufuhr von Luft in geeigneter Weise geregelt werden.

Die Schadstoffbelastung der zumindest einen bestimmten Person kann beispielsweise durch Emissionen von Pyrolyseprodukten, insbesondere Ozon, Kohlenstoffmonoxid, nitrose Gase, oder Feinstäuben, insbesondere Chrom-Verbindungen, Nickeloxid, Zinkoxid, Manganoxid, oder dgl. erfasst werden. Diese genannten Schadstoffe treten besonders bei Schweißprozessen auf und können ein gesundheitliches Risiko für die Schweißer darstellen, sofern bestimmte Konzentrationen überschritten werden.

Gelöst wird die erfindungsgemäße Aufgabe auch durch eine oben genannte Vorrichtung zur Erfassung der Schadstoffbelastung einer bestimmten Person in der Umgebung zumindest einer Bearbeitungsvorrichtung, wobei zumindest ein Sensor zur Ermittlung der Position der Person in Abhängigkeit der Zeit, weiters eine Datenbank zum Speichern von Schadstoffemissionen in Abhängigkeit der durchgeführten Bearbeitungsprozesse, und eine mit dem zumindest einen Sensor und der Datenbank verbundene Verarbeitungseinrichtung vorgesehen ist, welche Verarbeitungseinrichtung zur Abschätzung der auf die Person wirkenden Schadstoffemissionen in Abhängigkeit der ermittelten Position der Person und anhand der in Abhängigkeit des durchgeführten Bearbeitungsprozesses abgerufenen Schadstoffemissionen aus der Datenbank ausgebildet ist. Eine solche Vorrichtung ist relativ einfach und kostengünstig realisierbar, sofern die in der Datenbank gespeicherten Schadstoffemissionen verfügbar sind. Natürlich ist die Erhebung der Erfahrungswerte der Schadstoffemissionen in Abhängigkeit der durchgeführten Bearbeitungsprozesse mit einem erheblichen Aufwand verbunden. Zu den weiteren Vorteilen der Vorrichtung wird auf die obige Beschreibung des Verfahrens verwiesen.

Wie bereits oben erwähnt wurde, kann der zumindest eine Sensor zur Ermittlung der Position der Person durch einen an der Person angeordneten Sensor, beispielsweise einem Mobiltelefon oder eine Smartwatch, und bzw. oder durch einen in der Umgebung der Person angeordneten Sensor, beispielsweise eine Kamera, gebildet sein. Dadurch kann die Position der Person ohne großen Aufwand und ohne wesentliche Einschränkung der Person in ihrer Mobilität ermittelt werden.

Vorzugsweise ist mit der Verarbeitungseinrichtung eine Warnvorrichtung verbunden, um der Person und bzw. oder einer übergeordneten Stelle anzuzeigen, wenn ein Grenzwert der Schadstoffbelastung erreicht wurde. Die Warnvorrichtung kann zur Ausgabe einer optischen, akustischen oder taktilen Warnung ausgebildet sein. Die Warnung kann auch an den Grad der Annäherung oder Überschreitung von Schadstoffgrenzwerten angepasst werden und beispielsweise die Lautstärke einer akustischen Warnung oder die Farbe einer optischen Warnung entsprechend angepasst werden. Über die Warnung hinaus kann natürlich auch ein absoluter oder relativer Wert der Schadstoffbelastung bzw. einzelner Schadstoffemissionen auf einer geeigneten Anzeige wiedergegeben werden. Die Anzeige kann bei der bestimmten Person, an der Bearbeitungsvorrichtung oder auch einer übergeordneten Stelle angeordnet sein.

Wenn mit der Verarbeitungseinrichtung zumindest ein Lüfter zur Zufuhr von Frischluft und bzw. oder zur Luftzirkulation und bzw. oder zur Absaugung verbunden ist, kann in Abhängigkeit der abgeschätzten Schadstoffemissionen eine gezielte Verbesserung der Umgebungsluft der Bearbeitungsvorrichtungen erzielt werden.

Wenn mit der Verarbeitungseinrichtung zumindest ein an zumindest einer vorgegebenen Positionen angeordneter weiterer Sensor zur Messung der Schadstoffemissionen verbunden ist, können zur Erfassung der Schadstoffbelastung einer bestimmten Person zusätzlich zu den gespeicherten Schadstoffemissionen tatsächliche Schadstoffemissionen herangezogen werden. Dadurch können noch genauere Werte und verlässlichere Aussagen getroffen werden.

Wenn zusätzlich eine Einrichtung zur Überwachung der Position der zumindest einen Bearbeitungsvorrichtung vorgesehen und mit der Verarbeitungseinrichtung verbunden ist, kann ein allfälliger Positionswechsel der zumindest einen Bearbeitungsvorrichtung bei der Erfassung der Schadstoffbelastung berücksichtigt werden. Die Position der Bearbeitungsvorrichtungen wird vorzugsweise automatisch über geeignete Sensoren und Detektoren überwacht. Im einfachsten Fall kann die Position aber auch manuell in die Verarbeitungseinrichtung eingegeben und bei der Verarbeitung berücksichtigt werden.

Die vorliegende Erfindung wird anhand der beigefügten Figuren näher erläutert. Darin zeigen:
- Fig. 1: schematische Darstellung einer Bearbeitungsvorrichtung zur Bearbeitung von Werkstücken und in der Umgebung anwesender Personen;
- Fig. 2: ein Beispiel der auf eine bestimmte Person in der Umgebung zumindest einer Bearbeitungsvorrichtung wirkenden Schadstoffemissionen Abhängigkeit der Zeit;
- Fig. 3: ein anders Beispiel der auf eine bestimmte Person in der Umgebung zumindest einer Bearbeitungsvorrichtung wirkenden Schadstoffemissionen in Abhängigkeit längerer Zeiträume;
- Fig. 4: schematisch verschiedene Varianten der Ermittlung der Position einer Person in der Umgebung einer Bearbeitungsvorrichtung;
- Fig. 5: eine schematische Produktionshalle mit mehreren Bearbeitungsvorrichtungen und Personen in deren Umgebung; und
- Fig. 6A bis 6C: gespeicherte Schadstoffemissionen in Abhängigkeit verschiedener Eigenschaften der Bearbeitungsprozesse, nämlich in Abhängigkeit der Leistung der Bearbeitungsprozesse, in Abhängigkeit des Abstands der Person von der zumindest einen Bearbeitungsvorrichtung und in Abhängigkeit der Stabilität der Bearbeitungsprozesse.

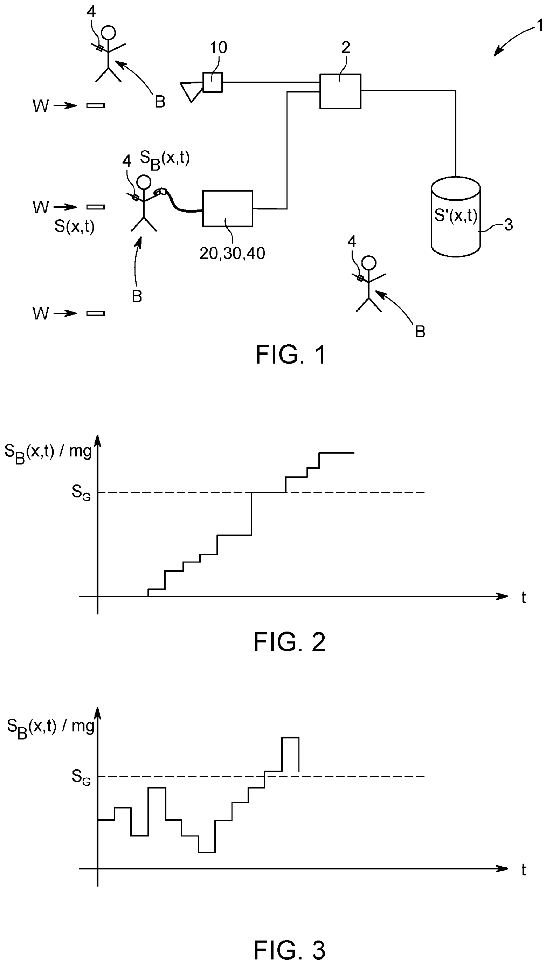

Fig. 1 zeigt eine schematische Darstellung einer Bearbeitungsvorrichtung 20 zur Bearbeitung von Werkstücken W und in der Umgebung anwesender Personen B. Im dargestellten Ausführungsbeispiel handelt es sich bei der Bearbeitungsvorrichtung 20 zur Ausführung von Bearbeitungsprozessen BPᵢ um eine Lichtbogenverarbeitungsvorrichtung 30 zur Ausführung eines Lichtbogenprozesses LP, vorzugsweise um eine Schweißvorrichtung 40 zur Ausführung eines Schweißprozesses SP. Es sind drei Werkstücke W dargestellt, die an verschiedenen Positionen innerhalb der Produktionshalle bzw. in der Umgebung der Bearbeitungsvorrichtung 20 angeordnet sind. Die zumindest eine Person B bearbeitet mit Hilfe der Bearbeitungsvorrichtung 20, insbesondere der Schweißvorrichtung 40 bzw. einem damit verbundenen Schweißbrenner das Werkstück W. Während des Schweißprozesses SP kommt es zu Schadstoffemissionen S(x,t), welche sowohl vom Ort x als auch von der Zeit t abhängen. Je nach Art der Bearbeitungsprozesse BPᵢ und je nach Position der Person B wirken bestimmte Schadstoffemissionen S_{B}(x,t) auf diese Person B. Diese auf die Personen B wirkenden Schadstoffemissionen S_{B}(x,t) können durch entsprechende Sensoren, welche an den Personen B, beispielsweise in einem Schweißschirm (nicht dargestellt) angeordnet sind, gemessen werden. Allerdings schränken diese Sensoren oder Schweißhelme die Person B ein und werden deswegen häufig nicht toleriert. Darüber hinaus sind in einer Produktionshalle üblicherweise auch weitere Personen B, welche nicht unbedingt mit der Bearbeitung der Werkstücke W beschäftigt sind und daher auch keine Schutzbekleidung tragen, anwesend, und ebenfalls zu einem gewissen Grad einer Schadstoffbelastung ausgesetzt. Ziel ist es daher, die Schadstoffbelastung aller Personen B, welche sich in der Umgebung der Bearbeitungsvorrichtungen 20 aufhalten, für jede Person B erfassen zu können, um deren allfällige Gesundheitsschädigung rechtzeitig erkennen zu können und entsprechende Gegenmaßnahmen setzen zu können.

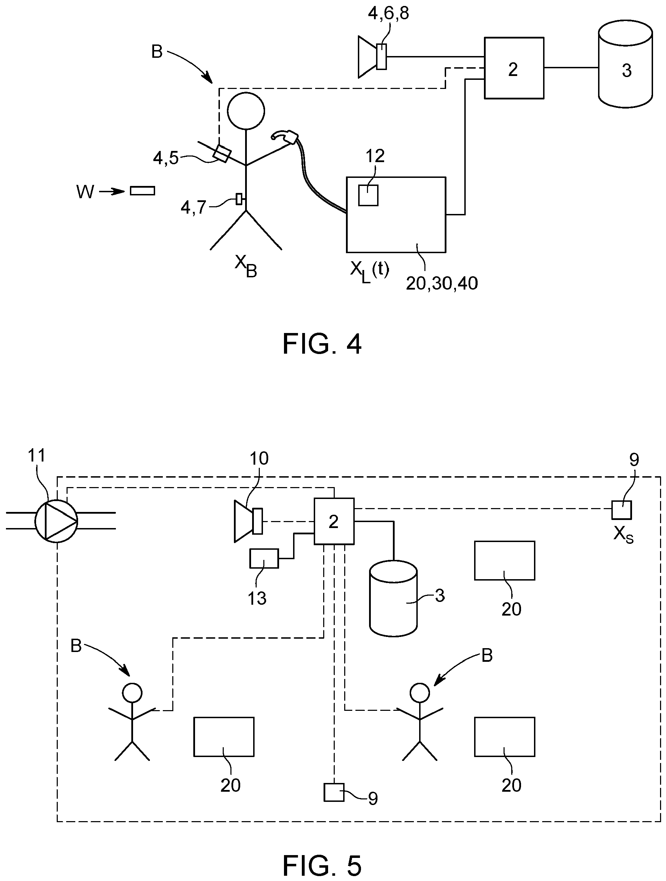

Zu diesem Zweck ist erfindungsgemäß eine Vorrichtung 1 zur Erfassung der Schadstoffbelastung einer bestimmten Person B durch aufgrund der Bearbeitungsprozesse BPᵢ auftretende Schadstoffemissionen Sᵢ(x,t) vorgesehen. Über zumindest einen Sensor 4 wird die Position x_{B}(t) der Person B in Abhängigkeit der Zeit t ermittelt. Dabei kann der Sensor 4 an der Person B oder in der Umgebung der Person B angeordnet sein (siehe Fig. 4). Weiters ist eine Datenbank 3 vorgesehen, in welcher Schadstoffemissionen Sᵢ' (x,t) bestimmter Schadstoffe in Abhängigkeit der durchgeführten Bearbeitungsprozesse BPᵢ gespeichert sind. Die Datenbank 3 enthält also Erfahrungswerte von Schadstoffemissionen Sᵢ' (x,t), wie sie bei den Bearbeitungsprozessen BPᵢ an den Werkstücken W üblicherweise auftreten. Beispielsweise kann für einen Schweißprozess SP an einem Werkstück W aus Aluminium bei Kenntnis der Leistung P, mit welchem der Schweißprozess SP durchgeführt wird, aus Erfahrungswerten geschlossen werden, wie viel Ozon, Feinstaub, Kohlenstoffmonoxid oder dgl. freigesetzt wird. Die Datenbank 3 und der zumindest eine Sensor 4 zur Ermittlung der Position x_{B}(t) der Person B in Abhängigkeit der Zeit t ist mit einer Verarbeitungseinrichtung 2 verbunden, in welcher die Verarbeitung der ermittelten Daten, also der Position x_{B}(t) der Person B und der Schadstoffemissionen Si' (x,t) in Abhängigkeit der durchgeführten Bearbeitungsprozesse BPᵢ stattfindet und daraus die auf die Person B wirkenden Schadstoffemissionen S_{B}(X,t) abgeschätzt werden. Wo die Verarbeitungseinrichtung 2 angeordnet ist, ist irrelevant. Die Verarbeitungseinrichtung 2 kann auch in der Ferne in einer Zentrale platziert sein und über entsprechende Datenverbindungen mit den Einrichtungen und Sensoren der Bearbeitungsvorrichtungen 20 verbunden sein oder auch in einer Bearbeitungsvorrichtung 20, beispielsweise einer Schweißvorrichtung 40, integriert sein.

Somit kann ohne aufwändige, insbesondere große und empfindliche, Sensoren an den Personen B oder in der Umgebung der Bearbeitungsvorrichtungen 20 eine relativ genaue und verlässliche Abschätzung der auf die Person B wirkenden Schadstoffbelastung S_{B}(x,t) durchgeführt werden. Über eine mit der Verarbeitungseinrichtung 2 verbundene Warnvorrichtung 10 kann eine akustische, optische oder auch taktile Warnung abgegeben werden, wenn vorbestimmte Grenzwerte an Schadstoffemissionen S_{B}(x,t) überschritten werden. Dann kann die Person B zum Einhalten einer Pause zum Regenerieren verpflichtet werden oder es können auch entsprechende Schritte zur Verbesserung der Luft in der Umgebung der Bearbeitungsvorrichtungen 20 gesetzt werden. Beispielsweise kann Abluft abgesaugt und Frischluft zugeführt werden, wobei hier jeweils Menge und Ort der abgesaugten und bzw. oder zugeführten Luft geregelt werden kann (siehe Fig. 5).

Fig. 2 zeigt ein Beispiel der auf eine bestimmte Person B in der Umgebung zumindest einer Bearbeitungsvorrichtung 20 wirkenden Schadstoffemissionen S_{B}(x,t) Abhängigkeit der Zeit t. Dementsprechend steigt die Konzentration der Schadstoffe im Laufe der Zeit t an. Beim Überschreiten vorgegebener Grenzwerte S_{G} der Schadstoffemissionen S_{B}(x,t) kann es zu einer Gesundheitsgefährdung der Person B kommen, weshalb das Überschreiten des Grenzwertes S_{G} vermieden werden muss.

In Fig. 3 ist ein anderes Beispiel, der auf eine bestimmte Person B in der Umgebung zumindest einer Bearbeitungsvorrichtung 20 wirkenden Schadstoffemissionen S_{B}(x,t) in Abhängigkeit längerer Zeiträume t, dargestellt. Durch Regeneration kann es auch zu einem Abfall der Schadstoffemissionen S_{B}(x,t) im Laufe der Zeit t kommen. Jedenfalls dürfen auch hier bestimmte Grenzwerte S_{G} an Schadstoffemissionen S_{B}(x,t) nicht überschritten werden, um die bestimmten Personen B gesundheitlich nicht zu gefährden.

Fig. 4 zeigt schematisch verschiedene Varianten der Ermittlung der Position x_{B}(t) einer Person B in der Umgebung zumindest einer Bearbeitungsvorrichtung 20, beispielsweise einer Lichtbogenbearbeitungsvorrichtung 30, vorzugsweise einer Schweißvorrichtung 40. Der zumindest eine Sensor 4 zur Ermittlung der Position x_{B}(t) der Person B kann durch einen an der Person B angeordneten Sensor 5, beispielsweise ein Mobiltelefon 7, gebildet sein. Alternativ oder zusätzlich kann die Position x_{B}(t) der Person B auch durch einen in der Umgebung der Person B angeordneten Sensor 6, beispielsweise eine Kamera 8, gebildet sein.

Über eine Einrichtung 12 kann zusätzlich die Position x_{L}(t) der zumindest einen Bearbeitungsvorrichtung 20 ermittelt und bei der Verarbeitung in der Verarbeitungseinrichtung 2 berücksichtigt werden.

In Fig. 5 ist eine schematische Produktionshalle mit mehreren Bearbeitungsvorrichtungen 20 und Personen B in deren Umgebung wiedergegeben. Zusätzlich zum Ausführungsbeispiel gemäß Fig. 1 ist hier an zumindest einer vorgegebenen Positionen x_{S} ein weiterer Sensor 9 zur Messung der tatsächlichen Schadstoffemissionen S(x,t) an dieser Position x_{S} angeordnet und mit der Verarbeitungseinrichtung 2 verbunden. Neben der Warnvorrichtung 10 ist auch eine Anzeige 13 zur Anzeige der auf die bestimmte Person B wirkenden Schadstoffemissionen S_{B}(x,t) mit der Verarbeitungseinrichtung 2 verbunden.

Wenn mit der Verarbeitungseinrichtung 2 zumindest ein Lüfter 11, insbesondere ein Ventilator, zur Zufuhr von Frischluft und bzw. oder zur Luftzirkulation und bzw. oder zur Absaugung verbunden ist, kann in Abhängigkeit der erfassten Schadstoffbelastungen auf die Personen B eine gezielte, insbesondere ortsabhängige und bzw. oder optimierte, Lüftung der Fertigungshalle durchgeführt werden.

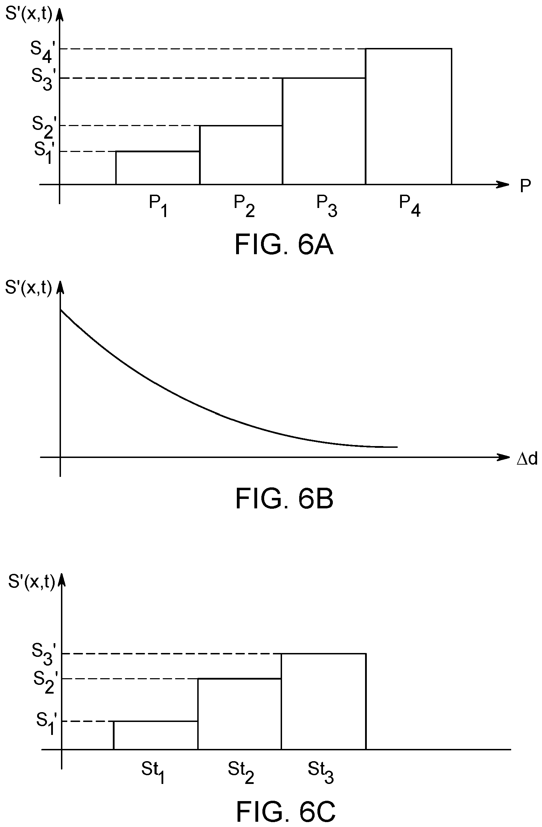

Schließlich zeigen die Fig. 6A bis 6C gespeicherte Schadstoffemissionen Si' (x,t) in Abhängigkeit verschiedener Eigenschaften der Bearbeitungsprozesse BPᵢ.

In Fig. 6A sind verschiedene Schadstoffemissionen S₁', S₂', S₃' und S₄' für verschiedene Leistungen P₁, P₂, P₃ und P₄ eines Bearbeitungsprozesses BPᵢ dargestellt. Je nach verwendeter Leistung Pᵢ des Bearbeitungsprozesses BPᵢ wird demnach der entsprechende gespeicherte Wert Sᵢ' für die Schadstoffemission S(x,t) für die Berechnung bzw. Abschätzung der auf die Person B wirkende Schadstoffemission S_{B}(x,t) herangezogen.

In Fig. 6B ist die gespeicherte Schadstoffemission S' (x,t) in Abhängigkeit des Abstands Δd der Person B von der zumindest einen Bearbeitungsvorrichtung 20 dargestellt. Dementsprechend nimmt die Schadstoffemission S' (x,t) exponentiell mit dem Abstand Δd zur Bearbeitungsvorrichtung 20 ab.

Schließlich zeigt Fig. 6C die gespeicherte Schadstoffemission S' (x,t) in Abhängigkeit der Stabilität Stᵢ der Bearbeitungsprozesse BPᵢ. Mit St₁ ist ein relativ stabiler Bearbeitungsprozess BP₁ dargestellt, bei dem die gespeicherte Schadstoffemission S₁' relativ gering ist. Bei einem weniger stabilen Bearbeitungsprozess BP₂ mit Stabilität St₂ sind die gespeicherten Schadstoffemissionen S₂' entsprechend höher als beim stabileren Bearbeitungsprozess BP₁. Als letztes ist ein instabiler Bearbeitungsprozess BP₃ mit Stabilität St₃ dargestellt. Die gespeicherten Schadstoffemission S₃' sind dementsprechend groß. Wird nun die Stabilität Stᵢ des Bearbeitungsprozesses BPᵢ ermittelt, kann die gespeicherte Schadstoffemission Si' (x,t) in Abhängigkeit der Stabilität Stᵢ des Bearbeitungsprozesses BPᵢ zur Abschätzung der auf die Person B wirkenden Schadstoffemission S_{B}(x,t) herangezogen werden.

Die vorliegende Erfindung schafft ein Verfahren und eine Vorrichtung zur Erfassung der Schadstoffbelastung einer bestimmten Person in der Umgebung zumindest einer Bearbeitungsvorrichtung, durch welche die auf die Person wirkenden Schadstoffemissionen in Abhängigkeit der ermittelten Position der Person und anhand der aus der Datenbank abgerufenen gespeicherten Schadstoffemissionen in Abhängigkeit der durchgeführten Bearbeitungsprozesse BPᵢ sehr gut abgeschätzt werden können. In der Folge können rechtzeitig vor dem Erreichen bestimmter Grenzwerte S_{G} für die Schadstoffbelastungen Warnungen ausgegeben werden und die Personen B zur Einhaltung von Pausen zur Regeneration aufgefordert werden. Aus den ermittelten Daten können Diagramme geschaffen werden, welchen Belastungen die Personen B während der Bearbeitungsprozesse BPᵢ ausgesetzt sind und daraus Schlüsse gezogen werden, wie die Bedingungen während der Bearbeitungsprozesse BPᵢ innerhalb einer Produktionshalle verbessert werden können.

Wie bereits oben erwähnt, können als bestimmte Personen B auch fiktive Personen B verstanden werden, deren Position x_{B}(t) innerhalb der Produktionshalle angenommen wird und deren fiktive Schadstoffbelastung mit dem beschriebenen Verfahren und der beschriebenen Vorrichtung erfasst wird. Auf diese Weise kann die Gefährlichkeit einer Produktionshalle vor dem Zutritt von Personen B abgeschätzt und können gegebenenfalls Maßnahmen ergriffen werden, um die Personen vor Gesundheitsgefährdungen zu schützen.

## Patentansprüche

1. Verfahren zur Erfassung der Schadstoffbelastung einer bestimmten Person (B) in der Umgebung zumindest einer Bearbeitungsvorrichtung (20) zur Durchführung von Bearbeitungsprozessen (BPᵢ) an Werkstücken (W) durch aufgrund der Bearbeitungsprozesse (BPᵢ) auftretende Schadstoffemissionen (Sᵢ(x,t)), **dadurch gekennzeichnet, dass** die Position (x_{B}(t)) der Person (B) in Abhängigkeit der Zeit (t) ermittelt wird, weiters in einer Datenbank (3) in Abhängigkeit der durchgeführten Bearbeitungsprozesse (BPᵢ) gespeicherte Schadstoffemissionen (Sᵢ' (x,t)) abgerufen werden und die Schadstoffbelastung der Person (B) dadurch erfasst wird, dass die auf die Person (B) wirkenden Schadstoffemissionen (S_{B}(x,t)) in einer Verarbeitungseinrichtung (2) in Abhängigkeit der ermittelten Position (x_{B}(t)) der Person (B) und anhand der aus der Datenbank (3) abgerufenen gespeicherten Schadstoffemissionen (Sᵢ' (x,t)) in Abhängigkeit der durchgeführten Bearbeitungsprozesse (BPᵢ) abgeschätzt werden.

2. Verfahren nach Anspruch 1, **dadurch gekennzeichnet, dass** die gespeicherten Schadstoffemissionen (Sᵢ' (x,t)) in Abhängigkeit des Materials der bearbeiteten Werkstücke (W) und bzw. oder der Art der Bearbeitungsprozesse (BPᵢ) und bzw. oder Parametern (Pᵢ) der Bearbeitungsprozesse (BPᵢ), beispielsweise der Leistung (Pᵢ) der Bearbeitungsprozesse (BPᵢ), und bzw. oder der Stabilität (Stᵢ) der Bearbeitungsprozesse (BPᵢ) abgerufen werden.

3. Verfahren nach Anspruch 1 oder 2, **dadurch gekennzeichnet, dass** die Position (x_{B}(t)) der Person (B) mit zumindest einem an der Person (B) angeordneten Sensor (5), beispielsweise einem Mobiltelefon (7), und bzw. oder mit zumindest einem in der Umgebung der Person (B) angeordneten Sensor (6), beispielsweise einer Kamera (8), ermittelt wird.

4. Verfahren nach einem der Ansprüche 1 bis 3, **dadurch gekennzeichnet, dass** die Position (x_{L}(t)) der zumindest einen Bearbeitungsvorrichtung (20) ermittelt und bei der Abschätzung der Schadstoffbelastung der Person (B) berücksichtigt wird.

5. Verfahren nach einem der Ansprüche 1 bis 4, **dadurch gekennzeichnet, dass** an zumindest einer vorgegebenen Position (x_{S}) mit zumindest einem weiteren Sensor (9) die Schadstoffemissionen (S(x,t)) gemessen und bei der Abschätzung der Schadstoffbelastung der Person (B) berücksichtigt werden.

6. Verfahren nach einem der Ansprüche 1 bis 5, **dadurch gekennzeichnet, dass** die Schadstoffbelastung der Person (B) erfasst wird, indem die auf die Person (B) wirkenden abgeschätzten Schadstoffemissionen (S_{B}(x,t)) mit der Zeit (t) aufsummiert werden.

7. Verfahren nach einem der Ansprüche 1 bis 6, **dadurch gekennzeichnet, dass** in Abhängigkeit der erfassten Schadstoffbelastung der Person (B) eine Warnung ausgegeben und bzw. oder gespeichert wird.

8. Verfahren nach einem der Ansprüche 1 bis 7, **dadurch gekennzeichnet, dass** in Abhängigkeit der erfassten Schadstoffbelastung der Person (B) zumindest ein Lüfter (11) zur Zufuhr von Frischluft und bzw. oder zur Luftzirkulation und bzw. oder zur Absaugung geregelt wird.

9. Verfahren nach einem der Ansprüche 1 bis 8, **dadurch gekennzeichnet, dass** die Schadstoffbelastung durch Emissionen von Pyrolyseprodukten, insbesondere Ozon, Kohlenstoffmonoxid, nitrose Gase, oder Feinstäuben, insbesondere Chrom-Verbindungen, Nickeloxid, Zinkoxid, Manganoxid, oder dgl. erfasst werden.

10. Vorrichtung (1) zur Erfassung der Schadstoffbelastung einer bestimmten Person (B) in der Umgebung zumindest einer Bearbeitungsvorrichtung (20) zur Durchführung von Bearbeitungsprozessen (BPᵢ) an Werkstücken (W) durch aufgrund der Bearbeitungsprozesse (BPᵢ) auftretende Schadstoffemissionen (Sᵢ(x,t)), **dadurch gekennzeichnet, dass** zumindest ein Sensor (4) zur Ermittlung der Position (x_{B}(t)) der Person (B) in Abhängigkeit der Zeit (t), weiters eine Datenbank (3) zum Speichern von Schadstoffemissionen (Sᵢ' (x,t)) in Abhängigkeit der durchgeführten Bearbeitungsprozesse (BPᵢ), und eine mit dem zumindest einen Sensor (4) und der Datenbank (3) verbundene Verarbeitungseinrichtung (2) vorgesehen ist, welche Verarbeitungseinrichtung (2) zur Abschätzung der auf die Person (B) wirkenden Schadstoffemissionen (S_{B}(x,t)) in Abhängigkeit der ermittelten Position (x_{B}(t)) der Person (B) und anhand der in Abhängigkeit der durchgeführten Bearbeitungsprozesse (BPᵢ) abgerufenen Schadstoffemissionen (Sᵢ' (x,t)) aus der Datenbank (3) ausgebildet ist.

11. Vorrichtung (1) nach Anspruch 10, **dadurch gekennzeichnet, dass** der zumindest eine Sensor (4) zur Ermittlung der Position (x_{B}(t)) der Person (B) durch einen an der Person (B) angeordneten Sensor (5), beispielsweise einem Mobiltelefon (7), und bzw. oder durch einen in der Umgebung der Person (B) angeordneten Sensor (6), beispielsweise eine Kamera (8), gebildet ist.

12. Vorrichtung (1) nach Anspruch 10 oder 11, **dadurch gekennzeichnet, dass** mit der Verarbeitungseinrichtung (2) eine Warnvorrichtung (10) verbunden ist.

13. Vorrichtung (1) nach einem der Ansprüche 10 bis 12, **dadurch gekennzeichnet, dass** mit der Verarbeitungseinrichtung (2) zumindest ein Lüfter (11) zur Zufuhr von Frischluft und bzw. oder zur Luftzirkulation und bzw. oder zur Absaugung verbunden ist.

14. Vorrichtung (1) nach einem der Ansprüche 10 bis 13, **dadurch gekennzeichnet, dass** mit der Verarbeitungseinrichtung (2) zumindest ein an zumindest einer vorgegebenen Positionen (x_{S}) angeordneter weiterer Sensor (9) zur Messung der Schadstoffemissionen (S(x,t)) verbunden ist.

15. Vorrichtung (1) nach einem der Ansprüche 10 bis 14, **dadurch gekennzeichnet, dass** eine Einrichtung (12) zur Überwachung der Position (x_{L}(t)) der zumindest einen Bearbeitungsvorrichtung (20) vorgesehen und mit der Verarbeitungseinrichtung (2) verbunden ist.
